# EUROPEAN PATENT SPECIFICATION

(11) **EP 4 235 572 B1**
(45) Date of publication and mention of the grant of the patent: **18.11.2026**
(21) Application number: 23158273.5
(22) Date of filing: 23.02.2023
(51) Int. Cl.: G06T 7/33

(54) **AUTOMATED REGISTRATION METHOD OF 3D FACIAL SCAN DATA AND 3D VOLUMETRIC MEDICAL IMAGE DATA USING DEEP LEARNING AND COMPUTER READABLE MEDIUM HAVING PROGRAM FOR PERFORMING THE METHOD**
AUTOMATISCHES REGISTRIERUNGSVERFAHREN FÜR 3D-GESICHTSSCANDATEN UND 3D-MEDIZINISCHE BILDDATEN MIT DEEP LEARNING UND COMPUTERLESBARES MEDIUM MIT PROGRAMM ZUR DURCHFÜHRUNG DES VERFAHRENS
MÉTHODE DE RECALAGE AUTOMATIQUE DE DONNÉES DE SCANNING FACIAL 3D ET DE DONNÉES D'IMAGES MÉDICALES VOLUMÉTRIQUES 3D UTILISANT L'APPRENTISSAGE PROFOND ET SUPPORT LISIBLE PAR ORDINATEUR COMPORTANT UN PROGRAMME POUR L'EXÉCUTION DE LA MÉTHODE

(30) Priority: 28.02.2022 KR 20220026434; 04.03.2022 WO PCT/KR2022/003132
(43) Date of publication of application: 30.08.2023
(73) Proprietor: Imagoworks Inc., Seoul 02455 (KR)
(72) Inventor: KIM, Hannah, Seoul (KR); SHIN, Bonjour, Seoul (KR); CHOI, Jinhyeok, Seoul (KR); KIM, Youngjun, Seoul (KR)
(74) Representative: BCKIP Part mbB

(56) References cited:
- WO-A1-2020/206135
- WO-A1-2021/125851
- US-A1- 2022 395 329
- KIM KYUNG-A ET AL: "Three-dimensional soft tissue changes according to skeletal changes after mandibular setback surgery by using cone-beam computed tomography and a structured light scanner", vol. 20, no. 1, December 2019 (2019-12-01), XP093060974, Retrieved from the Internet <URL:https://progressinorthodontics.springeropen.com/counter/pdf/10.1186/s40510-019-0282-0.pdf> DOI: 10.1186/s40510-019-0282-0
- RASMUS R PAULSEN ET AL: "Multi-view consensus CNN for 3D facial landmark placement", ARXIV.ORG, CORNELL UNIVERSITY LIBRARY, 201 OLIN LIBRARY CORNELL UNIVERSITY ITHACA, NY 14853, 14 October 2019 (2019-10-14), XP081514933, DOI: 10.1007/978-3-030-20887-5_44

## Description

### PRIORITY STATEMENT

This application claims priority on Korean Patent Application No. 10-2022-0026434, filed on February 28, 2022 in the Korean Intellectual Property Office (KIPO) and International Patent Application No. PCT/KR2022/003132 filed on March 4, 2022.

### TECHNICAL FIELD

The present invention relates to an automated registration method of three dimensional (3D) facial scan data and 3D volumetric medical image data using deep learning and a non-transitory computer-readable storage medium having stored thereon program instructions of the automated registration method of 3D facial scan data and 3D volumetric medical image data. More particularly, the present invention relates to an automated registration method of 3D facial scan data and 3D volumetric medical image data using deep learning and not requiring a user input or a conversion of a data structure and a non-transitory computer-readable storage medium having stored thereon program instructions of the automated registration method of 3D facial scan data and 3D volumetric medical image data.

### BACKGROUND

A 3D volumetric medical image data may refer to accumulated data of hundreds of two dimensional (2D) slide images such as computed tomography (CT) images, cone-beam CT (CBCT) images and magnetic resonance imaging (MRI) images. The 3D volumetric medical image data may include various tissues and structures of a body in a 3D form so that the 3D volumetric medical image data may be widely used for diagnosis, treatment and surgical planning in most medical fields including internal medicine and surgery. In dentistry, oral and maxillofacial surgery and plastic surgery, etc., the 3D volumetric medical image data for a head and neck region may be acquired and used to diagnose and treat a patient's maxillofacial and oral cavity.

The head and neck region may be an important body region because of aesthetic aspects as well as functional aspects such as breathing, mastication, and pronunciation. Therefore, both functional considerations and aesthetic preferences of a patient are reflected in maxillofacial and oral procedures and surgical plans. However, the 3D volumetric medical image data has intensity information of only a single channel and does not include information about colors of various tissues and structures of the body.

In addition, in order to three-dimensionally analyze a shape of a specific area, a process of segmenting the area or reconstructing the segmented data into a 3D model may be required. The process of segmenting the area and reconstructing the segmented data may require a lot of time. In addition, the 3D volumetric medical image data is expensive to acquire, and in the case of CT or CBCT, it is difficult to acquire multiple times due to the risk of radiation exposure. Therefore, it is difficult to compare the 3D volumetric medical image data before surgery and the 3D volumetric medical image data after surgery and it is difficult to use the 3D volumetric medical image data for procedures and surgical simulations in various facial expression states.

In order to compensate for these limitations of 3D volumetric medical image data, 3D facial scan mesh model data are being used with the 3D volumetric medical image data. The 3D volumetric medical image data may include texture, which is color information, as well as 3D shape information of the face shape and is inexpensive to acquire. A patient may not be exposed to radiation at all during the acquisition process of the 3D volumetric medical image data. Therefore, it is easy to compare the 3D volumetric medical image data before surgery and the 3D volumetric medical image data after surgery and it is easy to use the 3D volumetric medical image data for procedures and surgical simulations in various facial expression states.

In addition, when the 3D facial scan mesh model data are used with the 3D volumetric medical image data, it is helpful in establishing a procedure and surgical plan considering the aesthetic shape and color as well as the anatomical information of the inside of the head and neck region. However, since the 3D volumetric medical image data and the 3D facial scan mesh model data have different 3D spatial coordinate systems, a registration process of aligning and overlapping these two data must be preceded. However, a manual registration process in the 3D space requires skill and a lot of time.

Conventionally, methods for registration of 3D facial scan mesh model data and 3D volumetric medical image data have been proposed for this purpose. Since the 3D facial scan mesh model data and the 3D volumetric medical image data have different data structures, a process of unifying the different data structures into one was preceded in the conventional methods. The conventional methods are divided into following two methods: 1. a method of performing a 3D mesh-based registration after unifying a skin area into 3D mesh model data by segmentation and 3D reconstruction of 3D volumetric medical image data; 2. a method of performing an image-based registration by voxelizing the 3D facial scan mesh data into the 3D volumetric medical image data. However, the above mentioned segmentation and 3D reconstruction are time consuming tasks. In addition, when the 3D volumetric medical image data have a low image quality like CBCT, or the data have a heavy metal noise due to dental prosthesis or braces, the segmentation may be difficult so that the above mentioned method may not be applied.

In the case of a method of voxelization of the 3D facial scan mesh data, the shape information of the mesh data may be lost so that an accuracy of the registration may be decreased.

Furthermore, in another conventional method, matching points may be manually inputted to two different data and the matching points of the two different data may be overlapped. In another conventional method, markers may be attached to the patient, two different data having the markers may be acquired and a marker-based registration of the two different data may be performed. In the method of using the manual matching points, it is difficult to input accurate matching points, so that inconsistent results may be derived depending on a user or the same user's repetitive work. In the method of attaching the markers to the patient, the data must be acquired with the multiple markers attached to the patient so that the patient may experience discomfort. In addition, in the method of attaching the markers to the patient, a separate process of extracting markers through a user input or inputting marker positions may be required.

Document WO 2021/125851 A1 discloses a method in which a computer models a custom breast prosthesis, wherein the method comprises: acquiring medical image data and body scan data, 3D-modeling the medical image data so as to acquire first image data, 3D-modeling the body scan data so as to acquire second image data, merging the first image data and the second image data so as to generate third image data, and generating breast prosthesis appearance information on the basis of the third image data.

### SUMMARY OF THE INVENTION

It is one of the objects of the present invention to provide a fast automated registration method of 3D facial scan data and 3D volumetric medical image data using deep learning, not requiring a user input, an additional 3D model extraction process or a conversion of a data structure such as a voxelization, and utilizing the 3D facial scan data and the 3D volumetric medical image data itself.

A further object of the invention is to provide a non-transitory computer-readable storage medium having stored thereon program instructions of the automated registration method of 3D facial scan data and 3D volumetric medical image data.

To this end, the invention provides a method in accordance with claim 1, a method in accordance with claim 12, and a non-transitory computer-readable storage medium in accordance with claim 15.

A method of 3D facial scan data and 3D volumetric medical image data using deep learning according to the present invention includes extracting scan landmarks from the 3D facial scan data using a convolutional neural network, extracting volume landmarks from the 3D volumetric medical image data using the convolutional neural network and operating an initial registration of the 3D facial scan data and the 3D volumetric medical image data using the scan landmarks and the volume landmarks.

According to a first aspect of the invention, extracting the scan landmarks includes generating a first 2D depth image, which may represent a distance between a first reference plane disposed outside the 3D facial scan data and a face surface in the 3D facial scan data.

According to the first aspect of the invention, an input of the convolutional neural network is the first 2D depth image. An output of the convolutional neural network may be first 2D coordinates corresponding to the scan landmarks.

In an embodiment, the extracting scan landmarks may further include inverse-projecting the first 2D coordinates to first 3D coordinates based on a transformation method used when generating the first 2D depth image.

In an embodiment, an output of the convolutional neural network may be first 3D coordinates corresponding to the scan landmarks.

According to the first aspect of the invention, extracting the volume landmarks includes generating a second 2D depth image which may represent a distance between a second reference plane disposed outside the 3D volumetric medical image data and a face surface in the 3D volumetric medical image data.

In an embodiment, in the generating a second 2D depth image, while moving from a starting point of the 3D volumetric medical image data to an inside of the 3D volumetric medical image data, a position where a change in brightness value is greater than a threshold value firstly may be generated as a depth value of the second 2D depth image.

In an embodiment, in the generating a second 2D depth image, while moving from a starting point of the 3D volumetric medical image data to an inside of the 3D volumetric medical image data, a position where a change in brightness value is greater than a threshold value firstly and having a brightness value within a predetermined range of skin brightness values may be generated as a depth value of the second 2D depth image.

In an embodiment, an output of the convolutional neural network may be second 2D coordinates corresponding to the volume landmarks.

In an embodiment, the extracting volume landmarks may further include inverse-projecting the second 2D coordinates to second 3D coordinates based on a transformation method used when generating the second 2D depth image.

In an embodiment, an output of the convolutional neural network may be second 3D coordinates corresponding to the scan landmarks.

According to a second aspect of the invention, extracting scan landmarks includes generating a first 2D captured image by capturing the 3D facial scan data, wherein an input of the convolutional neural network is the first 2D captured image, and an output of the convolutional neural network is first 3D coordinates corresponding to the scan landmarks.

According to the second aspect of the invention, extracting volume landmarks includes generating a second 2D captured image by capturing the 3D volume medical image data, wherein an input of a convolutional neural network is the second 2D captured image. An output of the convolutional neural network is second 3D coordinates corresponding to the volume landmarks.

In an embodiment, a number of the scan landmarks extracted in the extracting scan landmarks may be equal to or greater than three. A number of the volume landmarks extracted in the extracting volume landmarks may be equal to or greater than three. The three or more scan landmarks may correspond to the three or more volume landmarks.

In an embodiment, the scan landmarks extracted in the extracting scan landmarks and the volume landmarks extracted in the extracting volume landmarks may include Soft tissue nasion, Right Exocanthion and Left Exocanthion respectively.

In an embodiment, the scan landmarks extracted in the extracting scan landmarks and the volume landmarks extracted in the extracting volume landmarks may include at least three among Soft tissue gabella, Soft tissue nasion, Supratip, Pronasale, Columella, Subnasale, Soft tissue A-point, Upper lip anterior point, Stomiom Superious, Lower point of gap between maxillary central incisors, Upper point of gap between mandibular central incisors, Stomiom Inferious, Lower lip anterior point, Soft tissue B-point, Soft tissue pogonion, Soft tissue gnathion, Soft tissue menton, Trichion, Right Orbitale superius, Left Orbitale superius, Right Palpebrale superius, Right Palpebrale inferious, Right Exocanthion, Right Endocanthion, Left Palpebrale superius, Left Palpebrale inferious, Left Endocanthion, Left Exocanthion, Right Zygion, Left Zygion, Right Cheilion, Left Cheilion, Right Tragus, Left Tragus, Right Soft tissue gonion, Left Soft tissue gonion, Right Alare, Left Alare, Right Alar curvature and Left Alar curvature respectively.

In an embodiment, the 3D facial scan data may be mesh data including 3D vertexes and triangle or rectangles generated by connecting the 3D vertexes.

In an embodiment, the 3D volumetric medical image data may be a medical image in which a patient's anatomy is obtained as 3D volumetric image data. The 3D volumetric medical image data may be one of a computed tomography (CT) image, a cone-beam CT (CBCT) image, a magnetic resonance imaging (MRI) image and a positron emission tomography (PET) image.

In an embodiment, the automated registration method may further include operating a fine registration after the operating an initial registration. In the fine registration, a distance between a plurality of sampling points in the 3D facial scan data and a plurality of sampling points in the 3D volumetric medical scan data may be adjusted to be less than a threshold distance.

According to an embodiment, the method includes extracting the scan landmarks from the 3D facial scan data using a first convolutional neural network, extracting the volume landmarks from the 3D volumetric medical image data using a second convolutional neural network different from the first convolutional neural network and operating an initial registration of the 3D facial scan data and the 3D volumetric medical image data using the scan landmarks and the volume landmarks.

According to an embodiment, extracting first scan landmarks from first 3D facial scan data using a convolutional neural network, extracting second scan landmarks from second 3D facial scan data using the convolutional neural network, extracting volume landmarks from the 3D volumetric medical image data using the convolutional neural network, operating an initial registration of the first 3D facial scan data, the second 3D facial scan data and the 3D volumetric medical image data using the first scan landmarks, the second scan landmarks and the volume landmarks and calculating a surface distance between the first 3D facial scan data and the second 3D facial scan data.

In an embodiment, the first 3D facial scan data and the second 3D facial scan data may differ in at least one of a scanning device, a scanning time, a facial expression, a shape of a portion of a face and coordinate axes.

A non-transitory computer-readable storage medium, according to a further aspect of the invention, has stored thereon program instructions, which when executed by at least one hardware processor, performs a method in accordance with the first or the second aspect of the invention.

According to the automated registration method of the 3D facial scan data and the 3D volumetric medical image data using deep learning, the registration of the 3D facial scan data and the 3D volumetric medical image data may be fast and automatically operated utilizing the 3D facial scan data and the 3D volumetric medical image data itself without requiring a user input, an additional 3D model extraction process or a conversion of a data structure such as a voxelization.

In addition, the user input and the conversion of the data structure may not be required so that the accuracy of the registration may be enhanced.

In addition, the 3D facial scan data acquired with various facial expressions and at various times may be automatically matched to the 3D volumetric medical image data so that the registration result of the 3D facial scan data and the 3D volumetric medical image data may be used for maxillofacial analysis of a patient and an analysis of before/after surgery of the patient.

In addition, when the landmarks of the 3D facial scan data and the landmarks of the 3D volumetric medical image data are extracted using the same convolutional neural network, the network structure may be simplified and the computing load for extracting the landmarks may be reduced.

### BRIEF DESCRIPTION OF THE DRAWINGS

The above and other features and advantages of the present inventive concept will become more apparent by describing in detailed embodiments thereof with reference to the accompanying drawings, in which:
FIG. 1 is a flowchart diagram illustrating an automated registration method of 3D facial scan data and 3D volumetric medical image data using deep learning according to an embodiment of the present invention;
FIG. 2 is a diagram illustrating an example of an operation of extracting scan landmarks from the 3D facial scan data of FIG. 1 and an example of an operation of extracting volume landmarks from the 3D volumetric medical image of FIG. 1;
FIG. 3 is a diagram illustrating an example of the operation of extracting the scan landmarks from the 3D facial scan data of FIG. 1 and an example of the operation of extracting the volume landmarks from the 3D volumetric medical image of FIG. 1;
FIG. 4 is a diagram illustrating a method of generating a first 2D depth image from the 3D facial scan data;
FIGS. 5 and 6 are diagrams illustrating a method of generating a second 2D depth image from the 3D volumetric medical image;
FIGS. 7 and 8 are diagrams illustrating examples of the scan landmarks and the volume landmarks;
FIG. 9 is a diagram illustrating an initial registration of FIG. 1;
FIG. 10 is a diagram illustrating a fine registration of FIG. 1;
FIG. 11 is a diagram illustrating a result of the fine registration of FIG. 1;
FIG. 12 is a diagram illustrating an example of an operation of extracting scan landmarks from the 3D facial scan data of FIG. 1 and an example of an operation of extracting volume landmarks from the 3D volumetric medical image of FIG. 1;
FIG. 13 is a diagram illustrating an example of an operation of extracting scan landmarks from the 3D facial scan data of FIG. 1 and an example of an operation of extracting volume landmarks from the 3D volumetric medical image of FIG. 1;
FIG. 14 is a diagram illustrating a first captured image generated by capturing the 3D facial scan data;
FIG. 15 is a diagram illustrating a second captured image generated by capturing the 3D volumetric medical image data;
FIG. 16 is a diagram illustrating an example of an automated registration of first 3D facial scan data, second 3D facial scan data and 3D volumetric medical image data; and
FIG. 17 is a diagram illustrating an example of an automated registration of first 3D facial scan data, second 3D facial scan data and 3D volumetric medical image data.

### DETAILED DESCRIPTION OF THE EMBODIMENTS

The present invention now will be described more fully hereinafter with reference to the accompanying drawings, in which embodiments of the present invention are shown. The present invention may, however, be embodied in many different forms and should not be construed as limited to the embodiments set forth herein.

Rather, these embodiments are provided so that this disclosure will be thorough and complete, and will fully convey the scope of the present invention to those skilled in the art. Like reference numerals refer to like elements throughout.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

The terminology used herein is for the purpose of describing particular embodiments only and is not intended to be limiting of the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the invention as used herein.

Hereinafter, the present invention will be explained in detail with reference to the accompanying drawings.

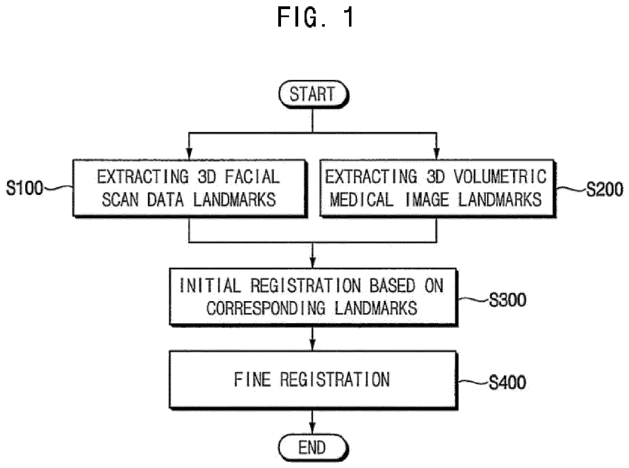

FIG. 1 is a flowchart diagram illustrating an automated registration method of 3D facial scan data and 3D volumetric medical image data using deep learning according to an embodiment of the present invention.

Referring to FIG. 1, the automated registration method of the 3D facial scan data and the 3D volumetric medical image data includes extracting scan landmarks from the 3D facial scan data using a convolutional neural network (operation S100), extracting volume landmarks from the 3D volumetric medical image data using the convolutional neural network (operation S200) and operating an initial registration of the 3D facial scan data and the 3D volumetric medical image data using the scan landmarks and the volume landmarks (operation S300). The automated registration method may further include operating a fine registration (operation S400) after operating the initial registration (operation S300). In the fine registration (operation S400), a distance between a plurality of sampling points in the 3D facial scan data and a plurality of sampling points in the 3D volumetric medical scan data may be adjusted to be less than a threshold distance.

The 3D facial scan data may be mesh data including 3D vertexes and triangle or rectangles generated by connecting the 3D vertexes. The 3D facial scan data may be image data captured through a 3D scanner. Alternatively, the 3D facial scan data may be a 3D facial model generated by reconstructing 2D facial image or a 3D facial template model generated using landmarks of the 2D facial image. A filename extension of the 3D facial scan data is not limited, and may be, for example, one of ply, obj and stl.

The 3D volumetric medical image data may be a medical image in which a patient's anatomy is obtained as 3D volumetric image data. The 3D volumetric medical image data may be one of a computed tomography (CT) image, a cone-beam CT (CBCT) image, a magnetic resonance imaging (MRI) image and a positron emission tomography (PET) image. In the present embodiment, a case in which the 3D volumetric medical image data is the computed tomography (CT) image will be described as an example.

For example, the number of the scan landmarks extracted in the operation of extracting the scan landmarks (operation S100) may be equal to or greater than three. For example, the number of the volume landmarks extracted in the operation of extracting the volume landmarks (operation S200) may be equal to or greater than three. The three or more scan landmarks may correspond to the three or more volume landmarks.

The automated registration method of the 3D facial scan data and the 3D volumetric medical image data of the present invention may be operated by a computing apparatus.

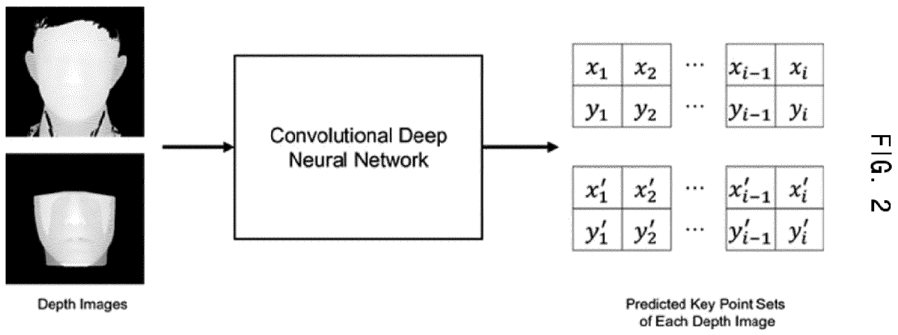

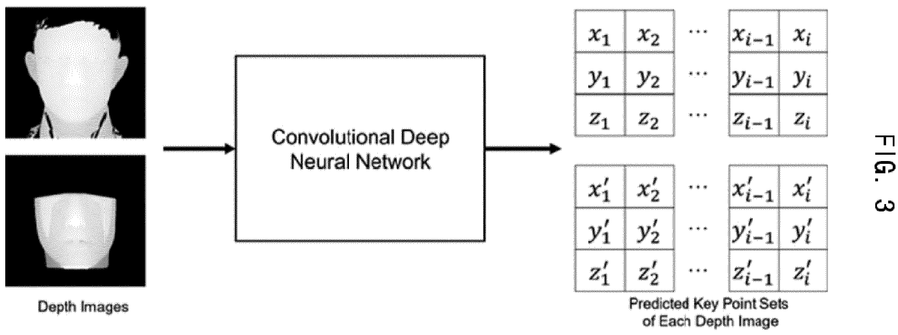

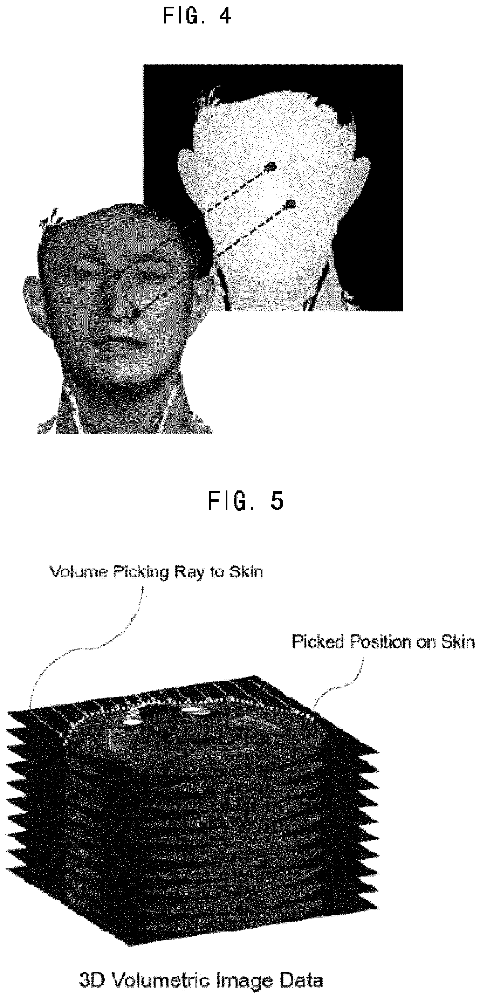

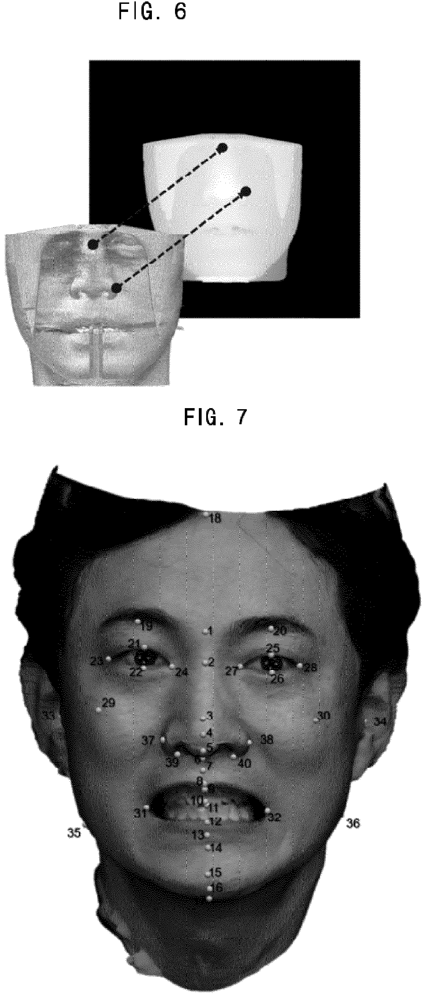

FIG. 2 is a diagram illustrating an example of an operation of extracting the scan landmarks from the 3D facial scan data of FIG. 1 and an example of an operation of extracting the volume landmarks from the 3D volumetric medical image of FIG. 1. FIG. 3 is a diagram illustrating an example of the operation of extracting the scan landmarks from the 3D facial scan data of FIG. 1 and an example of the operation of extracting the volume landmarks from the 3D volumetric medical image of FIG. 1. FIG. 4 is a diagram illustrating a method of generating a first 2D depth image from the 3D facial scan data. FIGS. 5 and 6 are diagrams illustrating a method of generating a second 2D depth image from the 3D volumetric medical image.

Referring to FIGS. 1 to 6, the operation of extracting the scan landmarks (operation S100) includes generating a first 2D depth image representing a distance between a first reference plane disposed outside the 3D facial scan data and a face surface in the 3D facial scan data. The first 2D depth image is illustrated in an upper left portion of FIG. 2.

FIG. 2 illustrates a case in which an input of the convolutional neural network is a 2D image and an output of the convolutional neural network is 2D coordinates.

According to the invention, the input of the convolutional neural network is the first 2D depth image. For example, the output of the convolutional neural network may be first 2D coordinates corresponding to the scan landmarks.

Herein, the operation of extracting the scan landmarks (operation S100) may further include inverse-projecting the first 2D coordinates to first 3D coordinates based on a transformation method used when generating the first 2D depth image. The first 3D coordinates may mean the landmarks of the 3D facial scan data.

Similarly, the operation of extracting the volume landmarks (operation S200) includes generating a second 2D depth image representing a distance between a second reference plane disposed outside the 3D volumetric medical image data and a face surface in the 3D volumetric medical image data. The second 2D depth image is illustrated in a lower left portion of FIG. 2.

According to the invention, the input of the convolutional neural network is the second 2D depth image. For example, the output of the convolutional neural network may be second 2D coordinates corresponding to the volume landmarks.

Herein, the operation of extracting the volume landmarks (operation S200) may further include inverse-projecting the second 2D coordinates to second 3D coordinates based on a transformation method used when generating the second 2D depth image. The second 3D coordinates may mean the landmarks of the 3D volumetric medical image data.

In FIG. 2, xᵢ,yᵢ represents the first 2D coordinates of an i-th scan landmark extracted from the 3D facial scan data and x'ᵢ,y'ᵢ represents the second 2D coordinates of an i-th volume landmark extracted from the 3D volumetric medical image data.

The scan landmark may be obtained by inverse-projecting the first 2D coordinates of FIG. 2 to the 3D facial scan data. The volume landmark may be obtained by inverse-projecting the second 2D coordinates of FIG. 2 to the 3D volumetric medical image data.

FIG. 3 illustrates a case in which an input of the convolutional neural network is a 2D image and an output of the convolutional neural network is 3D coordinates.

The input of the convolutional neural network is the first 2D depth image for the 3D facial scan data. For example, the output of the convolutional neural network may be first 3D coordinates corresponding to the scan landmarks.

In addition, the input of the convolutional neural network is the second 2D depth image for the 3D volumetric medical image data. For example, the output of the convolutional neural network may be second 3D coordinates corresponding to the volume landmarks.

In FIG. 3, *xᵢ,yᵢ,zᵢ* represents an i-th scan landmark extracted from the 3D facial scan data and *x'ᵢ,y'ᵢ,z'ᵢ* represents an i-th volume landmark extracted from the 3D volumetric medical image data.

In the present embodiment, the convolutional neural network for extracting the scan landmarks may be substantially the same as the convolutional neural network for extracting the volume landmarks. For example, the first 2D depth image of the 3D facial scan data and the second 2D depth image of the 3D volumetric medical image data may be inputted to the convolutional neural network. The first 2D coordinates or the first 3D coordinates corresponding to the scan landmarks and the second 2D coordinates or the second 3D coordinates corresponding to the volume landmarks may be outputted from the convolutional neural network.

Alternatively, the convolutional neural network for extracting the scan landmarks may be different from the convolutional neural network for extracting the volume landmarks. For example, the first 2D depth image of the 3D facial scan data may be inputted to a first convolutional neural network. The first 2D coordinates or the first 3D coordinates corresponding to the scan landmarks may be outputted from the first convolutional neural network. For example, the second 2D depth image of the 3D volumetric medical image data may be inputted to a second convolutional neural network which is different from the first convolutional neural network. The second 2D coordinates or the second 3D coordinates corresponding to the volume landmarks may be outputted from the second convolutional neural network.

FIG. 4 shows a process of generating a depth image form the 3D facial scan data. The depth image may be generated by representing a minimum distance from the surface of the 3D facial scan data to a virtual plane (the first reference plane) disposed at a certain distance from the 3D facial scan data as a brightness value (intensity). In FIG. 4, a lighter portion represents a shorter distance and a darker portion represents a longer distance. Alternatively, the brightness of the depth image may be inverted and represented in an opposite way.

FIGS. 5 and 6 show a process of obtaining a skin area from the 3D volumetric medical image data and generating a depth image using the obtained skin area. FIG. 5 shows a process of extracting a position of a skin surface where a brightness value (intensity) varies greatly when moving a virtual ray from a beginning to an end of the 3D volumetric medical image data and intersecting an air and the skin area into 3D coordinates. When a ray is irradiated at the beginning of the 3D volumetric medical image data, a first position where the brightness value changes greatly, and where the Hounsfield Unit (HU) value is between -100 and 300, which corresponds to skin and fat, may be considered as the skin area.

For example, in the operation of generating the second 2D depth image, while moving from a starting point of the 3D volumetric medical image data to an inside of the 3D volumetric medical image data, a position where a change in brightness value is greater than a threshold value firstly may be generated as a depth value of the second 2D depth image.

For example, in the operation of generating the second 2D depth image, while moving from a starting point of the 3D volumetric medical image data to an inside of the 3D volumetric medical image data, a position where a change in brightness value is greater than a threshold value firstly and having a brightness value within a predetermined range of skin brightness values may be generated as a depth value of the second 2D depth image. For example, the predetermined range of the skin brightness values may be Hounsfield Unit (HU) value between - 100 and 300.

The depth image may be generated by representing a minimum distance from the 3D coordinates of the extracted skin surface to a virtual plane (the second reference plane) disposed at a certain distance from the 3D volumetric medical image data as a brightness value (intensity). In FIG. 6, a lighter portion represents a shorter distance and a darker portion represents a longer distance. Alternatively, the brightness of the depth image may be inverted and represented in an opposite way.

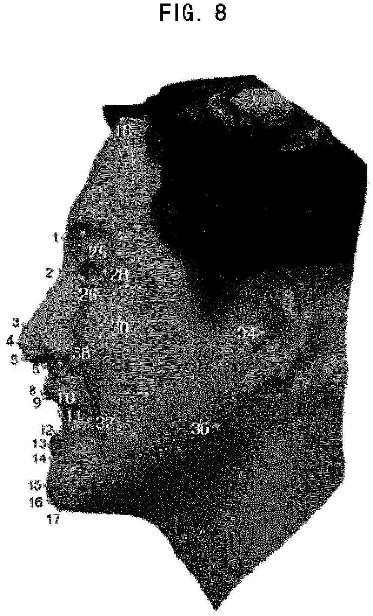

FIGS. 7 and 8 are diagrams illustrating examples of the scan landmarks and the volume landmarks.

Referring to FIGS. 1 to 8, the scan landmarks extracted in the operation (operation S100) of extracting the scan landmarks and the volume landmarks extracted in the operation (operation S200) of extracting the volume landmarks may include at least three among Soft tissue gabella(1), Soft tissue nasion(2), Supratip(3), Pronasale(4), Columella(5), Subnasale(6), Soft tissue A-point(7), Upper lip anterior point(8), Stomiom Superious(9), Lower point of gap between maxillary central incisors(10), Upper point of gap between mandibular central incisors(11), Stomiom Inferious(12), Lower lip anterior point(13), Soft tissue B-point(14), Soft tissue pogonion(15), Soft tissue gnathion(16), Soft tissue menton(17), Trichion(18), Right Orbitale superius(19), Left Orbitale superius(20), Right Palpebrale superius(21), Right Palpebrale inferious(22), Right Exocanthion(23), Right Endocanthion(24), Left Palpebrale superius(25), Left Palpebrale inferious(26), Left Endocanthion(27), Left Exocanthion(28), Right Zygion(29), Left Zygion(30), Right Cheilion(31), Left Cheilion(32), Right Tragus(33), Left Tragus(34), Right Soft tissue gonion(35), Left Soft tissue gonion(36), Right Alare(37), Left Alare(38), Right Alar curvature(39) and Left Alar curvature(40) respectively.

For example, the scan landmarks extracted in the operation (operation S100) of extracting the scan landmarks and the volume landmarks extracted in the operation (operation S200) of extracting the volume landmarks may include a skin surface landmark and an anterior tooth surface landmark.

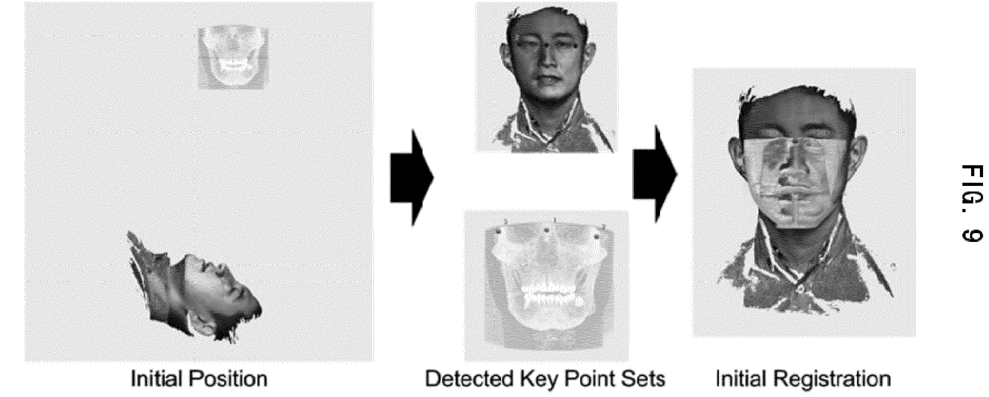

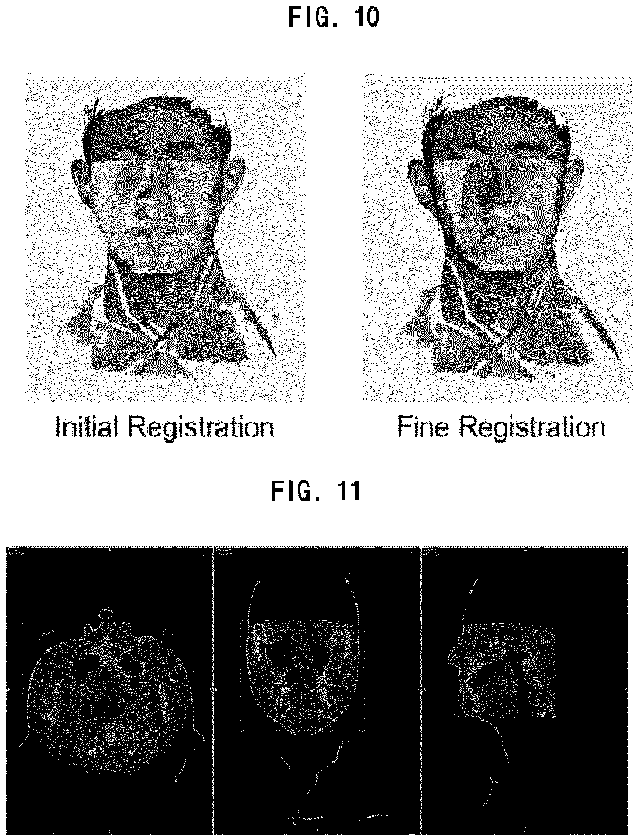

FIG. 9 is a diagram illustrating an initial registration (operation S300) of FIG. 1. FIG. 10 is a diagram illustrating a fine registration (operation S400) of FIG. 1. FIG. 11 is a diagram illustrating a result of the fine registration (operation S400) of FIG. 1.

Referring to FIGS. 1 to 11, in the initial registration (operation S300) which is based on the corresponding landmarks, a transform matrix M may be calculated by a landmark transform using at least three pairs of the landmarks extracted in the operations S100 and S200. In the initial registration (operation S300), an error may be calculated by calculating a distance difference between corresponding landmarks.

The transform matrix M may minimize a distance difference between corresponding landmarks and may move the scan landmarks of the 3D facial scan data to a domain of the 3D volumetric medical image data. FIG. 9 shows a result of the initial registration (operation S300) using the corresponding landmarks.

For example, the scan landmarks extracted in the operation (operation S100) of extracting the scan landmarks and the volume landmarks extracted in the operation (operation S200) of extracting the volume landmarks may include Soft tissue nasion, Right Exocanthion and Left Exocanthion respectively.

For example, the registration of the 3D facial scan data and the 3D volumetric medical image data may be operated using Soft tissue nasion, Right Exocanthion and Left Exocanthion in FIG. 10.

In the fine registration (operation S400), a precise registration may be performed to further match the 3D volumetric medical image and the 3D facial scan data after the initial registration (operation S300). FIGS. 10 and 11 show a result of the fine registration (operation S400).

As shown in FIG. 10, the 3D volumetric medical image data and the 3D facial scan data may be well matched in the result of the fine registration (operation S400) more than in the result of the initial registration (operation S300).

FIG. 11 visualizes the result of the fine registration (operation S400) as multi-view slide images of the 3D volumetric medical image data. The outer solid line in FIG. 11 corresponds to the skin surface of the 3D facial scan data. In FIG. 11, the outer solid line representing the skin surface of the 3D facial scan data is precisely matched to the 3D volumetric medical image data.

According to the present embodiment, the registration of the 3D facial scan data and the 3D volumetric medical image data may be fast and automatically operated utilizing the 3D facial scan data and the 3D volumetric medical image data itself without requiring a user input, an additional 3D model extraction process or a conversion of a data structure such as a voxelization.

In addition, the user input and the conversion of the data structure may not be required so that the accuracy of the registration may be enhanced.

In addition, when the landmarks of the 3D facial scan data and the landmarks of the 3D volumetric medical image data are extracted using the same convolutional neural network, the network structure may be simplified and the computing load for extracting the landmarks may be reduced.

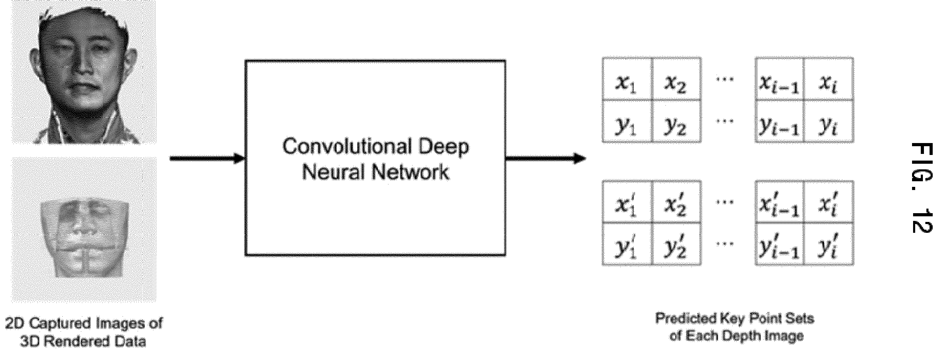

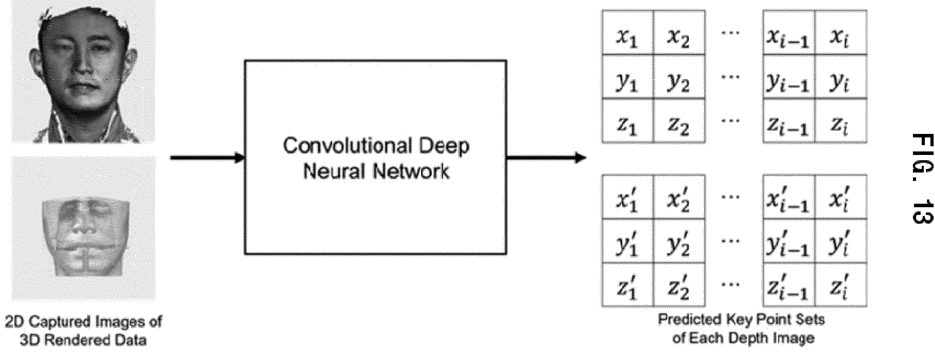

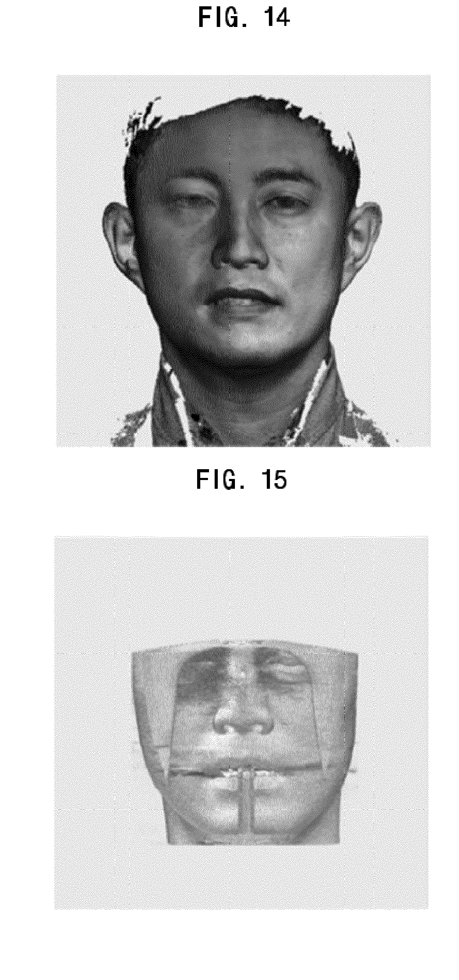

FIG. 12 is a diagram illustrating an example of an operation of extracting scan landmarks from the 3D facial scan data of FIG. 1 and an example of an operation of extracting volume landmarks from the 3D volumetric medical image of FIG. 1. FIG. 13 is a diagram illustrating an example of an operation of extracting scan landmarks from the 3D facial scan data of FIG. 1 and an example of an operation of extracting volume landmarks from the 3D volumetric medical image of FIG. 1. FIG. 14 is a diagram illustrating a first captured image generated by capturing the 3D facial scan data. FIG. 15 is a diagram illustrating a second captured image generated by capturing the 3D volumetric medical image data.

The automated registration method according to this aspect of the invention is substantially the same as the automated registration method of the previous embodiment explained referring to FIGS. 1 to 11 except for the input of the convolutional neural network. Thus, the same reference numerals will be used to refer to the same or like parts as those described in the previous embodiment of FIGS. 1 to 11 and any repetitive explanation concerning the above elements will be omitted.

Referring to FIGS. 1 and 7 to 15, the automated registration method of the 3D facial scan data and the 3D volumetric medical image data includes extracting scan landmarks from the 3D facial scan data using a convolutional neural network (operation S100), extracting volume landmarks from the 3D volumetric medical image data using the convolutional neural network (operation S200) and operating an initial registration of the 3D facial scan data and the 3D volumetric medical image data using the scan landmarks and the volume landmarks (operation S300). The automated registration method may further include operating a fine registration (operation S400) after operating the initial registration (operation S300). In the fine registration (operation S400), a distance between a plurality of sampling points in the 3D facial scan data and a plurality of sampling points in the 3D volumetric medical scan data may be adjusted to be less than a threshold distance.

In the operation of extracting the scan landmarks (operation S100), a first 2D captured image is generated by capturing the 3D facial scan data. The first 2D captured image is illustrated in an upper left portion of FIG. 12 and FIG. 14.

FIG. 12 illustrates a case in which an input of the convolutional neural network is a 2D image and an output of the convolutional neural network is 2D coordinates.

According to this aspect of the invention, the input of the convolutional neural network is the first 2D captured image, and the output of the convolutional neural network is first 2D coordinates corresponding to the scan landmarks.

Herein, the operation of extracting the scan landmarks (operation S100) may further include inverse-projecting the first 2D coordinates to first 3D coordinates. The first 3D coordinates may mean the landmarks of the 3D facial scan data.

Similarly, in the operation of extracting the volume landmarks (operation S200), a second 2D captured image is generated by capturing the 3D volumetric medical image data. The second 2D captured image is illustrated in a lower left portion of FIG. 12 and FIG. 15.

According to this aspect of the invention, the input of the convolutional neural network is the second 2D captured image, and the output of the convolutional neural network is second 2D coordinates corresponding to the volume landmarks.

Herein, the operation of extracting the volume landmarks (operation S200) may further include inverse-projecting the second 2D coordinates to second 3D coordinates. The second 3D coordinates may mean the landmarks of the 3D volumetric medical image data.

FIG. 13 illustrates a case in which an input of the convolutional neural network is a 2D image and an output of the convolutional neural network is 3D coordinates.

The input of the convolutional neural network is the first 2D captured image for the 3D facial scan data. The output of the convolutional neural network is first 3D coordinates corresponding to the scan landmarks.

In addition, the input of the convolutional neural network is the second 2D captured image for the 3D volumetric medical image data. For example, the output of the convolutional neural network may be second 3D coordinates corresponding to the volume landmarks.

According to the present embodiment, the registration of the 3D facial scan data and the 3D volumetric medical image data may be fast and automatically operated utilizing the 3D facial scan data and the 3D volumetric medical image data itself without requiring a user input, an additional 3D model extraction process or a conversion of a data structure such as a voxelization.

In addition, the user input and the conversion of the data structure may not be required so that the accuracy of the registration may be enhanced.

In addition, when the landmarks of the 3D facial scan data and the landmarks of the 3D volumetric medical image data are extracted using the same convolutional neural network, the network structure may be simplified and the computing load for extracting the landmarks may be reduced.

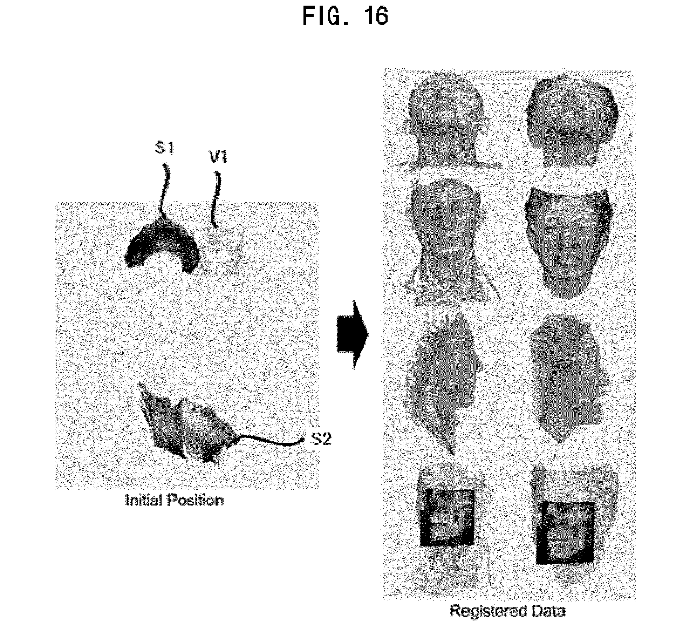

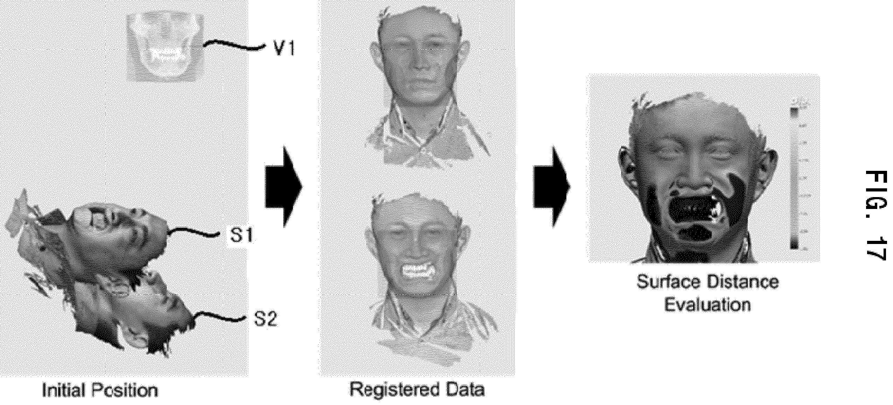

FIG. 16 is a diagram illustrating an example of an automated registration of first 3D facial scan data S1, second 3D facial scan data S2 and 3D volumetric medical image data V1. FIG. 17 is a diagram illustrating an example of an automated registration of first 3D facial scan data S1, second 3D facial scan data S2 and 3D volumetric medical image data V1.

The automated registration method according to the present embodiment is substantially the same as the automated registration method of the previous embodiment explained referring to FIGS. 1 to 11 except that two 3D facial scan data are matched to one 3D volumetric medical image data. Thus, the same reference numerals will be used to refer to the same or like parts as those described in the previous embodiment of FIGS. 1 to 11 and any repetitive explanation concerning the above elements will be omitted.

Referring to FIGS. 1 to 17, the automated registration method of the 3D facial scan data and the 3D volumetric medical image data includes extracting first scan landmarks from first 3D facial scan data S1 using a convolutional neural network, extracting second scan landmarks from second 3D facial scan data S2 using the convolutional neural network, extracting volume landmarks from the 3D volumetric medical image data V1 using the convolutional neural network, operating an initial registration of the first 3D facial scan data S1, the second 3D facial scan data S2 and the 3D volumetric medical image data V1 using the first scan landmarks, the second scan landmarks and the volume landmarks and calculating a surface distance between the first 3D facial scan data S1 and the second 3D facial scan data S2.

For example, the first scan landmarks, the second scan landmarks and the volume landmarks may be extracted from the same convolutional neural network.

Alternatively, the first scan landmarks and the second scan landmarks may be extracted from a first convolutional neural network and the volume landmarks may be extracted from a second convolutional neural network.

For example, the first 3D facial scan data S1 and the second 3D facial scan data S2 may differ in at least one of a scanning device, a scanning time, a facial expression, a shape of a specific portion of the face and coordinate axes.

FIG. 16 illustrates a case in which two 3D facial scan data S1 and S2 of the same patient with different scanning devices, different facial expressions and different coordinate axes are matched to one 3D volumetric medical image data V1.

FIG. 17 illustrates a case in which two 3D facial scan data S1 and S2 of the same patient with the same scanning device, but with different facial expressions and different coordinate axes are matched to one 3D volumetric medical image data V1.

FIG. 17 illustrates an analysis of a surface distance difference by calculating the surface distance between the first 3D facial scan data S1 and the second 3D facial scan data S2.

As shown in FIGS. 16 and 17, the 3D facial scan data S1 and S2 having different coordinate axes are matched to the same 3D volumetric medical image data V1 and the coordinate axes of the 3D facial scan data S1 and S2 coincide with each other so that the first 3D facial scan data S1 and the second 3D facial scan data S2 may be compared and analyzed.

In FIG. 16, two 3D facial scan data of the same patient with different scanning devices, different scanning times and different facial expressions are matched to one 3D volumetric medical image data so that the two 3D facial scan data may be compared and analyzed in various angles. In FIG. 17, two 3D facial scan data of the same patient with different facial expressions are matched to one 3D volumetric medical image data so that a face change area may be analyzed.

The 3D volumetric medical image data is expensive to acquire, and in the case of CT or CBCT, it is difficult to acquire multiple times due to the risk of radiation exposure. Therefore, it is difficult to compare the 3D volumetric medical image data before surgery and the 3D volumetric medical image data after surgery and it is difficult to use the 3D volumetric medical image data for procedures and surgical simulations in various facial expression states. In the present embodiment, the 3D facial scan data acquired with various facial expressions and at various times may be automatically matched to the 3D volumetric medical image data so that the registration result of the 3D facial scan data and the 3D volumetric medical image data may be used for maxillofacial analysis of a patient and an analysis of before/after surgery of the patient.

According to the present invention, the registration of the 3D facial scan data and the 3D volumetric medical image data may be fast and automatically operated utilizing the 3D facial scan data and the 3D volumetric medical image data itself without requiring a user input, an additional 3D model extraction process or a conversion of a data structure such as a voxelization.

In addition, the user input and the conversion of the data structure may not be required so that the accuracy of the registration may be enhanced.

In addition, when the landmarks of the 3D facial scan data and the landmarks of the 3D volumetric medical image data are extracted using the same convolutional neural network, the network structure may be simplified and the computing load for extracting the landmarks may be reduced.

According to an embodiment of the present invention, a non-transitory computer-readable storage medium having stored thereon program instructions of the automated registration method of 3D facial scan data and 3D volumetric medical image data is provided. The above mentioned method may be written as a program executed on the computer. The method may be implemented in a general purpose digital computer which operates the program using a computer-readable medium. In addition, the structure of the data used in the above mentioned method may be written on a computer readable medium through various means. The computer readable medium may include program instructions, data files and data structures alone or in combination. The program instructions written on the medium may be specially designed and configured for the present invention, or may be generally known to a person skilled in the computer software field. For example, the computer readable medium may include a magnetic medium such as a hard disk, a floppy disk and a magnetic tape, an optical recording medium such as CD-ROM and DVD, a magneto-optical medium such as floptic disc and a hardware device specially configured to store and execute the program instructions such as ROM, RAM and a flash memory. For example, the program instructions may include a machine language codes produced by a compiler and high-level language codes which may be executed by a computer using an interpreter or the like. The hardware device may be configured to operate as one or more software modules to perform the operations of the present invention.

In addition, the above mentioned automated registration method of 3D facial scan data and 3D volumetric medical image data may be implemented in a form of a computer-executed computer program or an application which are stored in a storage method.

The present invention is related to the automated registration method of 3D facial scan data and 3D volumetric medical image data and the non-transitory computer-readable storage medium having stored thereon program instructions of the automated registration method of 3D facial scan data and 3D volumetric medical image data. According to the present invention, the time and the effort for the automated registration. In addition, the 3D facial scan data acquired with various facial expressions and at various times may be automatically matched to the 3D volumetric medical image data so that the registration result of the 3D facial scan data and the 3D volumetric medical image data may be used for maxillofacial analysis of a patient and an analysis of before/after surgery of the patient.

The foregoing is illustrative of the present invention and is not to be construed as limiting thereof. Although a few embodiments of the present invention have been described, those skilled in the art will readily appreciate that many modifications are possible in the embodiments without materially departing from the scope of the present invention as defined in the claims.

## Claims

1. An automated registration method of 3D facial scan data and 3D volumetric medical image data using deep learning, the method comprising:
extracting scan landmarks from the 3D facial scan data using a convolutional neural network receiving as input a first 2D depth image generated from the 3D facial scan data;
extracting volume landmarks from the 3D volumetric medical image data using a convolutional neural network receiving as input a second 2D depth image generated from the 3D volumetric medical image data; and
operating an initial registration of the 3D facial scan data and the 3D volumetric medical image data using the scan landmarks and the volume landmarks.

2. The method of claim 1, wherein the first 2D depth image represents a distance between a first reference plane disposed outside the 3D facial scan data and a face surface in the 3D facial scan data.

3. The method of claim 2, wherein an output of the convolutional neural network is first 2D coordinates corresponding to the scan landmarks.

4. The method of claim 3, wherein extracting the scan landmarks further comprises:
inverse-projecting the first 2D coordinates to first 3D coordinates based on a transformation method used when generating the first 2D depth image.

5. The method of claim 2, wherein an output of the convolutional neural network is first 3D coordinates corresponding to the scan landmarks.

6. The method of any one of claims 1 to 5, wherein the second 2D depth image represents a distance between a second reference plane disposed outside the 3D volumetric medical image data and a face surface in the 3D volumetric medical image data.

7. The method of claim 6, wherein, in generating a second 2D depth image, while moving from a starting point of the 3D volumetric medical image data to an inside of the 3D volumetric medical image data, a position where a change in brightness value is greater than a threshold value firstly is generated as a depth value of the second 2D depth image.

8. The method of claim 6, wherein, in generating a second 2D depth image, while moving from a starting point of the 3D volumetric medical image data to an inside of the 3D volumetric medical image data, a position where a change in brightness value is greater than a threshold value firstly and having a brightness value within a predetermined range of skin brightness values is generated as a depth value of the second 2D depth image.

9. The method of any one of claims 6 to 8, wherein an output of the convolutional neural network is second 2D coordinates corresponding to the volume landmarks.

10. The method of claim 9, wherein extracting the volume landmarks further comprises:
inverse-projecting the second 2D coordinates to second 3D coordinates based on a transformation method used when generating the second 2D depth image.

11. The method of any one of claims 6 to 8, wherein an output of the convolutional neural network is second 3D coordinates corresponding to the scan landmarks.

12. An automated registration method of 3D facial scan data and 3D volumetric medical image data using deep learning, the method comprising:
extracting scan landmarks from the 3D facial scan data using a convolutional neural network receiving as input a first 2D captured image generated by capturing the 3D facial scan data, wherein an output of the convolutional neural network is first 2D or 3D coordinates corresponding to the scan landmarks,
extracting volume landmarks from the 3D volumetric medical image data using a convolutional neural network receiving as input a second 2D captured image generated by capturing the 3D volume medical image data, wherein an output of the convolutional neural network is second 2D or 3D coordinates corresponding to the volume landmarks; and
operating an initial registration of the 3D facial scan data and the 3D volumetric medical image data using the scan landmarks and the volume landmarks.

13. The method of any one of claims 1 to 12, wherein a number of the scan landmarks extracted in the extracting scan landmarks is equal to or greater than three,
wherein a number of the volume landmarks extracted in the extracting volume landmarks is equal to or greater than three,
wherein the three or more scan landmarks correspond to the three or more volume landmarks, and
wherein the scan landmarks extracted in the extracting scan landmarks and the volume landmarks extracted in the extracting volume landmarks include Soft tissue nasion, Right Exocanthion and Left Exocanthion respectively.

14. The method of any one of claims 1 to 13, further comprising:
operating a fine registration after the operating an initial registration,
wherein in the fine registration, a distance between a plurality of sampling points in the 3D facial scan data and a plurality of sampling points in the 3D volumetric medical scan data is adjusted to be less than a threshold distance.

15. A non-transitory computer-readable storage medium having stored thereon program instructions, which when executed by at least one hardware processor, performs the method of one of claims 1 to 14.

## Patentansprüche

1. Automatisiertes Verfahren zur Registrierung von 3D-Gesichtsscandaten und volumetrischen medizinischen 3D-Bilddaten unter Verwendung von Deep Learning, wobei das Verfahren Folgendes umfasst:
Extrahieren von Scan-Landmarken aus den 3D-Gesichtsscandaten unter Verwendung eines neuronalen Convolutional-Netzwerks, das als Eingabe ein erstes 2D-Tiefenbild empfängt, das aus den 3D-Gesichtsscandaten erzeugt wird;
Extrahieren von Volumen-Landmarken aus den volumetrischen medizinischen 3D-Bilddaten unter Verwendung eines neuronalen Convolutional-Netzwerks, das als Eingabe ein zweites 2D-Tiefenbild empfängt, das aus den volumetrischen medizinischen 3D-Bilddaten erzeugt wird; und
Durchführen einer anfänglichen Registrierung der 3D-Gesichtsscandaten und der volumetrischen medizinischen 3D-Bilddaten unter Verwendung der Scan-Landmarken und der Volumen-Landmarken.

2. Verfahren nach Anspruch 1, wobei das erste 2D-Tiefenbild einen Abstand zwischen einer ersten Referenzebene, die außerhalb der 3D-Gesichtsscandaten angeordnet ist, und einer Gesichtsoberfläche in den 3D-Gesichtsscandaten darstellt.

3. Verfahren nach Anspruch 2, wobei eine Ausgabe des neuronalen Convolutional-Netzwerks erste 2D-Koordinaten sind, die den Scan-Landmarken entsprechen.

4. Verfahren nach Anspruch 3, wobei das Extrahieren der Scan-Landmarken ferner Folgendes umfasst:
Rückprojizieren der ersten 2D-Koordinaten in erste 3D-Koordinaten auf der Grundlage eines Transformationsverfahrens, das beim Erzeugen des ersten 2D-Tiefenbilds verwendet wird.

5. Verfahren nach Anspruch 2, wobei eine Ausgabe des neuronalen Convolutional-Netzwerks erste 3D-Koordinaten sind, die den Scan-Landmarken entsprechen.

6. Verfahren nach einem der Ansprüche 1 bis 5, wobei das zweite 2D-Tiefenbild einen Abstand zwischen einer zweiten Referenzebene, die außerhalb der volumetrischen medizinischen 3D-Bilddaten angeordnet ist, und einer Gesichtsoberfläche in den volumetrischen medizinischen 3D-Bilddaten darstellt.

7. Verfahren nach Anspruch 6, wobei beim Erzeugen eines zweiten 2D-Tiefenbilds während einer Bewegung von einem Ausgangspunkt der volumetrischen medizinischen 3D-Bilddaten in das Innere der volumetrischen medizinischen 3D-Bilddaten eine Position, an der eine Änderung des Helligkeitswerts erstmals größer als ein Schwellenwert ist, als ein Tiefenwert des zweiten 2D-Tiefenbilds erzeugt wird.

8. Verfahren nach Anspruch 6, wobei beim Erzeugen eines zweiten 2D-Tiefenbilds während einer Bewegung von einem Ausgangspunkt der volumetrischen medizinischen 3D-Bilddaten in das Innere der volumetrischen medizinischen 3D-Bilddaten eine Position, an der eine Änderung des Helligkeitswerts erstmals größer als ein Schwellenwert ist und die einen Helligkeitswert innerhalb eines vorbestimmten Bereichs von Hauthelligkeitswerten aufweist, als ein Tiefenwert des zweiten 2D-Tiefenbilds erzeugt wird.

9. Verfahren nach einem der Ansprüche 6 bis 8, wobei eine Ausgabe des neuronalen Convolutional-Netzwerks zweite 2D-Koordinaten sind, die den Volumen-Landmarken entsprechen.

10. Verfahren nach Anspruch 9, wobei das Extrahieren der Volumen-Landmarken ferner Folgendes umfasst:
Rückprojizieren der zweiten 2D-Koordinaten in zweite 3D-Koordinaten auf der Grundlage eines Transformationsverfahrens, das beim Erzeugen des zweiten 2D-Tiefenbilds verwendet wird.

11. Verfahren nach einem der Ansprüche 6 bis 8, wobei eine Ausgabe des neuronalen Convolutional-Netzwerks zweite 3D-Koordinaten sind, die den Scan-Landmarken entsprechen.

12. Automatisiertes Verfahren zur Registrierung von 3D-Gesichtsscandaten und volumetrischen medizinischen 3D-Bilddaten unter Verwendung von Deep Learning, wobei das Verfahren Folgendes umfasst:
Extrahieren von Scan-Landmarken aus den 3D-Gesichtsscandaten unter Verwendung eines neuronalen Convolutional-Netzwerks, das als Eingabe ein erstes erfasstes 2D-Bild empfängt, das durch Erfassen der 3D-Gesichtsscandaten erzeugt wird, wobei eine Ausgabe des neuronalen Convolutional-Netzwerks erste 2D- oder 3D-Koordinaten sind, die den Scan-Landmarken entsprechen,
Extrahieren von Volumen-Landmarken aus den volumetrischen medizinischen 3D-Bilddaten unter Verwendung eines neuronalen Convolutional-Netzwerks, das als Eingabe ein zweites erfasstes 2D-Bild empfängt, das durch Erfassen der medizinischen 3D-Volumenbilddaten erzeugt wird, wobei eine Ausgabe des neuronalen Convolutional-Netzwerks zweite 2D- oder 3D-Koordinaten sind, die den Volumen-Landmarken entsprechen; und
Durchführen einer anfänglichen Registrierung der 3D-Gesichtsscandaten und der volumetrischen medizinischen 3D-Bilddaten unter Verwendung der Scan-Landmarken und der Volumen-Landmarken.

13. Verfahren nach einem der Ansprüche 1 bis 12, wobei eine Anzahl der beim Extrahieren der Scan-Landmarken extrahierten Scan-Landmarken gleich oder größer als drei ist,
wobei eine Anzahl der beim Extrahieren der Volumen-Landmarken extrahierten Volumen-Landmarken gleich oder größer als drei ist,
wobei die drei oder mehr Scan-Landmarken den drei oder mehr Volumen-Landmarken entsprechen, und
wobei die beim Extrahieren der Scan-Landmarken extrahierten Scan-Landmarken und die beim Extrahieren der Volumen-Landmarken extrahierten Volumen-Landmarken jeweils das Weichteil-Nasion, das rechte Exocanthion und das linke Exocanthion umfassen.

14. Verfahren nach einem der Ansprüche 1 bis 13, ferner umfassend:
Durchführen einer Feinregistrierung nach dem Durchführen einer anfänglichen Registrierung,
wobei bei der Feinregistrierung ein Abstand zwischen einer Vielzahl von Abtastpunkten in den 3D-Gesichtsscandaten und einer Vielzahl von Abtastpunkten in den volumetrischen medizinischen 3D-Scandaten so angepasst wird, dass er kleiner als ein Schwellenabstand ist.

15. Nichttransitorisches computerlesbares Speichermedium, auf dem Programmanweisungen gespeichert sind, die, wenn sie durch zumindest einen Hardwareprozessor ausgeführt werden, das Verfahren nach einem der Ansprüche 1 bis 14 durchführen.

## Revendications

1. Procédé automatisé de recalage de données de numérisation faciale 3D et de données d'images médicales volumétriques 3D utilisant l'apprentissage profond, le procédé comprenant :
l'extraction de points de repère de numérisation à partir des données de numérisation faciale 3D à l'aide d'un réseau neuronal convolutif recevant en entrée une première image de profondeur 2D générée à partir des données de numérisation faciale 3D ;
l'extraction de points de repère de volume à partir des données d'images médicales volumétriques 3D à l'aide d'un réseau neuronal convolutif recevant en entrée une seconde image de profondeur 2D générée à partir des données d'images médicales volumétriques 3D ; et
la réalisation d'un recalage initial des données de numérisation faciale 3D et des données d'images médicales volumétriques 3D à l'aide des points de repère de numérisation et des points de repère de volume.

2. Procédé selon la revendication 1, dans lequel la première image de profondeur 2D représente une distance entre un premier plan de référence disposé à l'extérieur des données de numérisation faciale 3D et une surface faciale dans les données de numérisation faciale 3D.

3. Procédé selon la revendication 2, dans lequel une sortie du réseau neuronal convolutif est constituée de premières coordonnées 2D correspondant aux points de repère de numérisation.

4. Procédé selon la revendication 3, dans lequel l'extraction des points de repère de numérisation comprend en outre :
la rétroprojection des premières coordonnées 2D en premières coordonnées 3D sur la base d'une méthode de transformation utilisée lors de la génération de la première image de profondeur 2D.

5. Procédé selon la revendication 2, dans lequel une sortie du réseau neuronal convolutif est constituée de premières coordonnées 3D correspondant aux points de repère de numérisation.

6. Procédé selon l'une quelconque des revendications 1 à 5, dans lequel la seconde image de profondeur 2D représente une distance entre un second plan de référence disposé à l'extérieur des données d'images médicales volumétriques 3D et une surface faciale dans les données d'images médicales volumétriques 3D.

7. Procédé selon la revendication 6, dans lequel, lors de la génération d'une seconde image de profondeur 2D, pendant un déplacement depuis un point de départ des données d'images médicales volumétriques 3D vers l'intérieur des données d'images médicales volumétriques 3D, une position où une variation de la valeur de luminosité est pour la première fois supérieure à une valeur seuil est générée en tant que valeur de profondeur de la seconde image de profondeur 2D.

8. Procédé selon la revendication 6, dans lequel, lors de la génération d'une seconde image de profondeur 2D, pendant un déplacement depuis un point de départ des données d'images médicales volumétriques 3D vers l'intérieur des données d'images médicales volumétriques 3D, une position où une variation de la valeur de luminosité est pour la première fois supérieure à une valeur seuil et qui présente une valeur de luminosité dans une plage prédéterminée de valeurs de luminosité de la peau est générée en tant que valeur de profondeur de la seconde image de profondeur 2D.

9. Procédé selon l'une quelconque des revendications 6 à 8, dans lequel une sortie du réseau neuronal convolutif est constituée de secondes coordonnées 2D correspondant aux points de repère de volume.

10. Procédé selon la revendication 9, dans lequel l'extraction des points de repère de volume comprend en outre :
la rétroprojection des secondes coordonnées 2D en secondes coordonnées 3D sur la base d'une méthode de transformation utilisée lors de la génération de la seconde image de profondeur 2D.

11. Procédé selon l'une quelconque des revendications 6 à 8, dans lequel une sortie du réseau neuronal convolutif est constituée de secondes coordonnées 3D correspondant aux points de repère de numérisation.

12. Procédé automatisé de recalage de données de numérisation faciale 3D et de données d'images médicales volumétriques 3D utilisant l'apprentissage profond, le procédé comprenant :
l'extraction de points de repère de numérisation à partir des données de numérisation faciale 3D à l'aide d'un réseau neuronal convolutif recevant en entrée une première image 2D capturée générée par capture des données de numérisation faciale 3D, dans lequel une sortie du réseau neuronal convolutif est constituée de premières coordonnées 2D ou 3D correspondant aux points de repère de numérisation,
l'extraction de points de repère de volume à partir des données d'images médicales volumétriques 3D à l'aide d'un réseau neuronal convolutif recevant en entrée une seconde image 2D capturée générée par capture des données d'images médicales de volume 3D, dans lequel une sortie du réseau neuronal convolutif est constituée de secondes coordonnées 2D ou 3D correspondant aux points de repère de volume ; et
la réalisation d'un recalage initial des données de numérisation faciale 3D et des données d'images médicales volumétriques 3D à l'aide des points de repère de numérisation et des points de repère de volume.

13. Procédé selon l'une quelconque des revendications 1 à 12, dans lequel un nombre de points de repère de numérisation extraits lors de l'extraction des points de repère de numérisation est égal ou supérieur à trois,
dans lequel un nombre de points de repère de volume extraits lors de l'extraction des points de repère de volume est égal ou supérieur à trois,
dans lequel les trois points de repère de numérisation ou plus correspondent aux trois points de repère de volume ou plus, et
dans lequel les points de repère de numérisation extraits lors de l'extraction des points de repère de numérisation et les points de repère de volume extraits lors de l'extraction des points de repère de volume comprennent respectivement le nasion des tissus mous, l'exocanthion droit et l'exocanthion gauche.

14. Procédé selon l'une quelconque des revendications 1 à 13, comprenant en outre :
la réalisation d'un recalage fin après la réalisation d'un recalage initial,
dans lequel, lors du recalage fin, une distance entre une pluralité de points d'échantillonnage dans les données de numérisation faciale 3D et une pluralité de points d'échantillonnage dans les données de numérisation médicale volumétrique 3D est ajustée de manière à être inférieure à une distance seuil.

15. Support de stockage non transitoire lisible par ordinateur, sur lequel sont stockées des instructions de programme qui, lorsqu'elles sont exécutées par au moins un processeur matériel, mettent en œuvre le procédé selon l'une des revendications 1 à 14.
